# EUROPEAN PATENT SPECIFICATION

(11) **EP 1 509 172 B1**
(45) Date of publication and mention of the grant of the patent: **04.09.2013**
(21) Application number: 03729109.3
(22) Date of filing: 23.05.2003
(51) Int. Cl.: A61F 2/14

(54) **PHOTOABLATABLE CORNEA INLAYS**
DURCH PHOTOABLATION MODIFIZIERBARE KORNEALE EINLAGELINSE
LENTICULES INTRA-CORNEENS PHOTOABLATIFS

(30) Priority: 31.05.2002 US 161394
(43) Date of publication of application: 02.03.2005
(73) Proprietor: BAUSCH & LOMB INCORPORATED, Rochester, New York 14604-2701 (US)
(72) Inventor: RUSCIO, Dominic, V., Webster, NY 14580 (US); KUNZLER, Jay, F., Canandaigua, NY 14424 (US); HOFFMANN, Laurent, G., Aliso Viejo, CA 92656 (US)
(74) Representative: Vossius & Partner
(86) International application number: PCT/US2003/016380
(87) International publication number: WO 2003/101348

(56) References cited:
- WO-A-01/37031
- WO-A-01/70335
- WO-A-91/16865
- WO-A-98/48715
- WO-A-99/07309
- US-A- 5 941 874
- US-B1- 6 197 019

## Description

### Field of the Invention

The present invention relates to photoablatable inlays (PAls). Herein described are also soft, optically transparent, hydrogel materials particularly suited for use in the production of PAls, and a method for manufacturing and using the same.

### Background of the Invention

Laser-assisted *in situ* keratomileusis (LASIK) surgery is a surgical refractive vision correction procedure that is extremely popular due in part to the relative lack of pain immediately following surgery and in part to the excellent vision usually achieved by the very next day, if not before. In LASIK surgery, a microkeratome is used to create a thin, circular flap in the cornea tissue of an eye. The surgeon folds the tissue flap out of the way, then removes corneal tissue and reshapes the cornea underneath the flap using an excimer laser. The tissue flap is then laid back in place, covering the area where the corneal tissue was shaped and removed. The major drawback of LASIK surgery is that the procedure is not reversible and additional surgeries are limited by the residual corneal thickness. Additional stromal material must be ablated for additional correction in the likely event a patient's vision deteriorates with time following LASIK surgery. Additional stromal material may not be present to accommodate such additional correction. Accordingly, a surgical refractive vision correction procedure, which is reversible and allows for additional surgeries over time as a patient's vision naturally deteriorates over time, is desired.

### Summary of the Invention

Soft, foldable, hydrogel polymeric materials having relatively high water contents particularly suited for use as photoablatable inlays (PAIs), corneal inlays, corneal onlays or like ophthalmic devices have now been discovered. The subject hydrogel polymeric materials are suitable for manufacture in the form of a disc or lenticule for placement in the corneal bed of an eye following surgical formation of a flap therein. The hydrogel polymeric material disc or lenticule is then precisely custom ablated *in situ* to the desired shape using an excimer laser. A surgical procedure using a PAI of the present invention is advantageous in that the number of corrective procedures is not limited by the thickness of the cornea. Likewise, a surgical procedure using a PAI of the present invention is reversible and repeatable to correct hyperopia, astigmatism, and mild to moderate myopia simply by ablating the PAI or replacing a former PAI with a new one that is then ablated to conform to the patient's specific needs.

WO 98/48715 describes a blank, adaptable for use in modifying the curvature of a patient's live cornea, comprising a first surface adapted for placement directly on a surface of said patient's live cornea; a second surface adapted to be exposed to a laser beam; a wall surface, extending between said first and second surfaces, and defining an opening in said blank; said blank comprising a material whose properties permit light having a wavelength within the visible spectrum to pass therethrough and prevent substantially all light having a wavelength within the laser light spectrum from passing therethrough, wherein said material is one of collagen, copolymer collagen, polyethylene oxide and hydrogel.

WO 99/07309 describes an intracorneal lens, comprising a) a base lens made from a first hydrogel material and having a refractive index greater than the refractive index of corneal tissue; b) a diffractive surface formed on the base, lens; and c) a coating covering the diffractive surface, the coating made from a second hydrogel material having a refractive index that is less than the refractive index of the first material.,

Accordingly, it is an object of the present invention to provide a biocompatible polymeric material.

Another object of the present invention is to provide a hydrogel polymeric material having a high water content similar to that of the cornea.

Another object of the present invention is to provide a hydrogel polymeric material that is colorless.

Another object of the present invention is to provide a hydrogel polymeric material that is transparent.

Another object of the present invention is to provide a polymeric material that is suitable for *in situ* photoablation.

Still another object of the present invention is to provide a biocompatible polymeric material that is relatively simple to manufacture.

These and other objectives and advantages of the present invention, some of which are specifically described and others that are not, will become apparent from the detailed description and claims that follow.

The present invention relates to a photo ablatable cornea inlay as described in claims 1 to 12.

### Detailed Description of the Invention

The following detailed description is provided to enable any person skilled in the art to which the present invention pertains to make and use the same, and sets forth the best mode contemplated by the inventors of carrying out the subject invention.

Herein described are also soft, optically transparent, hydrogel polymeric materials particularly suited for use in the production of PAls, and a method for manufacturing and using the same. The hydrogel polymeric materials used in the present invention maximize water content for use in the manufacture of ophthalmic devices such as photoablatable inlays (PAIs).

The preferred water content of subject hydrogel polymeric materials for improved biocompatability is approximately 78 percent, which is the reported water content of the human cornea. The subject hydrogel polymeric materials are manufactured in the form of a disc or lenticule for placement in the corneal bed of an eye following surgical formation of a flap therein. The hydrogel polymeric material disc or lenticule is suitable for precise custom ablation *in situ* to the desired shape using an excimer laser. A surgical procedure using a PAI of the present invention is advantageous in that the number of corrective procedures is not limited by the thickness of the particular patient's cornea as is true of LASIK surgical visual correction. Likewise, a surgical procedure using a PAI of the present invention is reversible and repeatable to correct hyperopia, astigmatism, and mild to moderate myopia simply by ablating the PAI or replacing a former PAI with a new one that is then ablated to meet the patient's specific needs.

The hydrogel polymeric materials used in the present invention are copolymers of hydrophilic monomers. Suitable hydrophilic monomers for use in the present invention are selected from the group consisting of 2-hydroxyethyl methacrylate, hydroxyethoxyethyl methacrylate, hydroxydiethoxyethyl methacrylate, methoxyethyl methacrylate, methoxyethoxyethyl methacrylate, methoxydiethoxyethyl methacrylate, poly(ethylene glycol) methacrylate, methoxy-poly(ethylene glycol) methacrylate, methacrylic acid, sodium methacrylate, glycerol methacrylate, hydroxypropyl methacrylate, N-vinylpyrrolidione, hydroxypropyl methacrylamide, N,N-dimethylacrylamide, N-methylacrylamide and hydroxybutyl methacrylate. Preferred hydrophilic monomers are 2-hydroxyethyl methacrylate (HEMA) and methacrylic acid (MAA) to maximize water content.

Hydrogel polymeric materials of the present invention include for example but are not limited to poly(2-hydroxyethyl methacrylate-co-methacrylic acid), poly(2-hydroxyethyl methacrylate-co-N-vinylpyrrolidinone), poly(2-hydroxyethyl methacrylate-co-dimethylacrylamide), poly(N-vinylpyrrolidinone -co-2-methacrylic acid), poly(2-hydroxyethyl methacrylate-co-4-t-butyl-2-hydroxyethyl methacrylate) and poly(N-vinylpyrrolidinone-co-4-t-butyl-2-hydroxyethyl methacrylate).

The subject hydrogel polymeric materials are synthesized by polymerizing one or more of the above-described hydrophilic monomers in the presence of 0.01 but more preferably 0.01 to 3.0 weight percent crosslinker and at least 0.01 but more preferably 0.02 to 2.0 weight percent initiator. Optionally, an ultraviolet light absorber may also be added.

Suitable crosslinkers include for example but are not limited to ethylene glycol dimethacrylate, diethylene glycol dimethacrylate, triethylene glycol dimethacrylate and poly(ethylene glycol) dimethacrylate wherein ethylene glycol dimethacrylate is preferred.

The hydrophilic monomers used in the present invention may be readily cured in cast shapes by one or more conventional methods. Such methods include for example but are not limited to ultraviolet light (UV) polymerization, visible light polymerization, microwave polymerization, thermal polymerization, free radical polymerization, living radical polymerization or combinations thereof. Metallocene catalysts may also be used in certain instances.

Suitable free radical thermal polymerization initiators include for example but are not limited to organic peroxides, such as acetyl peroxide, lauroyl peroxide, decanoyl peroxide, stearoyl peroxide, benzoyl peroxide, t-butyl peroxypivalate, peroxydicarbonate, and the like.

Representative UV initiators include those known in the field such as for example but not limited to benzoin methyl ether, benzoin ethyl ether, Darocur™ 1173, 1164, 2273, 1116, 2959 and 3331 (EM Industries, Inc., Hawthorne, New York) and Irgacur™ 651 and 184 (Ciba-Geigy, Basel, Switzerland).

used in

Other suitable initiators include for example but are not limited to azobis(isobutyronitrile), 2,2'-azobis(2,4-dimethylyaleronitrile), 2,2'-azobis(methylbutyronitrile), 1,1'-azobis(cyanocycloh exane), di-t-butyl peroxide, dicumyl peroxide, t-butylcumyl peroxide, 2,5-dimethyl-2,5-bis(2-ethylhexanoyl peroxy)hexane, t-butyl peroxyneodecanote, t-butyl peroxy 2-ethylhexanoate, di(4-t-butyl cyclohexyl) peroxydicarbonate, t-butyl peroxypivalate, decanoyl peroxide, lauroyl peroxide, benzoyl peroxide, 2.4-pentanedione peroxide, di(n-propyl) peroxydicarbonate, t-amyl peroxyneodecanoate and t-butyl peroxyacetate wherein 2,2'-azobis(isobutyronitrile) is preferred.

Suitable ultraviolet light absorbers include for example but are not limited to beta-(4-benzotriazoyl-3-hydroxyphenoxy)ethyl acrylate, 4-(2-acryloxyethoxy)-2-hydroxybenzophenone. 4-methacryloxy-2-hydroxybenzophenone, 2-(2'-methacryloxy-5'-methylphenyl)benzotriazole, 2-(2'-hydroxy-5'-methacryoxyethylphenyl)-2H-benzotriazole, 2-[3'-tert-butyl-2'-hydroxy-5'-(3"-methacryloyloxypropyl)phenyl]-5-chlorobenzotriazole, 2-[3'-tert-butyl-5'-(3"-dimethylvinylsilylpropoxy)-2'-hydroxyphenyl]-5-methoxybenzotriazole, 2-(3'-allyl-2'-hydroxy-5-methylphenyl)benzotriazole, 2-[3'-tert-butyl-2'-hydroxy-5'-(3"methacryloyloxypropoxy)phenyl]-5-methoxybenzotriazole, and 2-[3'-tert-butyl-2'-hydroxy-5'-(3"-methacryloyloxypropoxy)phenyl]-5-chlorobenzotriazole wherein beta-(4-benzotriazoyl-3-hydroxyphenoxy)ethyl acrylate is the preferred ultraviolet light absorber.

The subject hydrogel polymeric materials having a refractive index of approximately 1.30 or greater in the hydrated state as measured by an Abbe refractometer at 589 nm and 37 degrees Celsius with a sodium light source, and approximately 60 percent or greater, but preferably 65 to 90 percent and most preferably 70 to 80 percent water content by weight are described in still greater detail in the examples that follow.

### Example 1: Preparation of Acrylic-based Cast Molded Film (Sample 1)

HEMA (98 weight percent) and methacrylic acid (MAA) (2 weight percent) were combined in flasks. Enough EGDMA crosslinker was added to comprise 0.16 weight percent of total weight of HEMA and MAA. Darocur™ 1173 initiator was added to equal 0.5 weight percent of the total weight of monomers. The solution was cast in films by pouring the solution onto plates and exposing the same to ultraviolet radiation for two hours under nitrogen. Following ultraviolet radiation exposure, the films were annealed at 115° Celsius for fifteen minutes and then slowly cooled. Films having a thickness of approximately 560 µm were obtained. Discs or lenticules were then cut from the films for study.

### Example 2: Preparation of Acrylic-based Cast Molded Film (Sample 2)

HEMA (96 weight percent) and methacrylic acid (MAA) (4 weight percent) were combined in flasks. Darocur™ 1173 initiator was added to equal 0.5 weight percent of the total weight of monomers. The solution was cast in films by pouring the solution onto plates and exposing the same to ultraviolet radiation for two hours under nitrogen. Following ultraviolet radiation exposure, the films were annealed at 115° Celsius for fifteen minutes and then slowly cooled. Films having a thickness of approximately 560 µm were obtained. Discs or lenticules were then cut from the films for study.

### Example 3: Ablation Study of Acrylic-based Cast Molded Films:

A Visx™ excimer laser (Visx, Incorporated, Santa Clara, California) was used to do three phototherapeutic keratectomy (PTK) ablations of 25, 50 and 100 µm depths at usual clinical settings of 160 mJ and 10 Hz on sample discs or lenticules from Example 1 and Example 2 above, hereinafter referred to as Sample 1 and Sample 2, respectively. Prior to ablating the hydrated samples, Samples 1 and 2 were blotted to remove any excess surface moisture present from storage. Following ablation, Samples 1 and 2 were packaged in 5 ml vials with borate buffer and observed on a Nikon™ stereomicroscope (Nikon, Corporation, Japan) with a Nikon™ 950 digital camera and a SmartScope™/ROI microscope (Optical Gaging Products, Inc., Rochester, New York). Pictures and dimensions were taken. The ablation depths were measured on the SmartScope™/ROI microscope at x132 magnification under bright field conditions.

Ablation rates as a ratio of the measured ablation depth versus the intended ablation depth were measured for Samples 1 and 2. The ablation data is summarized in Table 1 below.

**Table 1**

| **Ablation Data Summary** | | | | | |
|---|---|---|---|---|---|
| **Sample Number** | **Water Content (%)** | **Measured vs. Intended Depth** | **Avg. Depth PTK @** | | |
| | | | **25 µm** | **50 µm** | **100 µm** |
| 1 | 73.1 | 4.01/1 | 121+5 | 224+5 | 422+10 |
| 2 | 82.3 | 4.62/1 | 141+5 | 256+5 | 488+10 |

The ablation data of Table 1 is likewise depicted in the graphs of Charts 1 and 2 below.

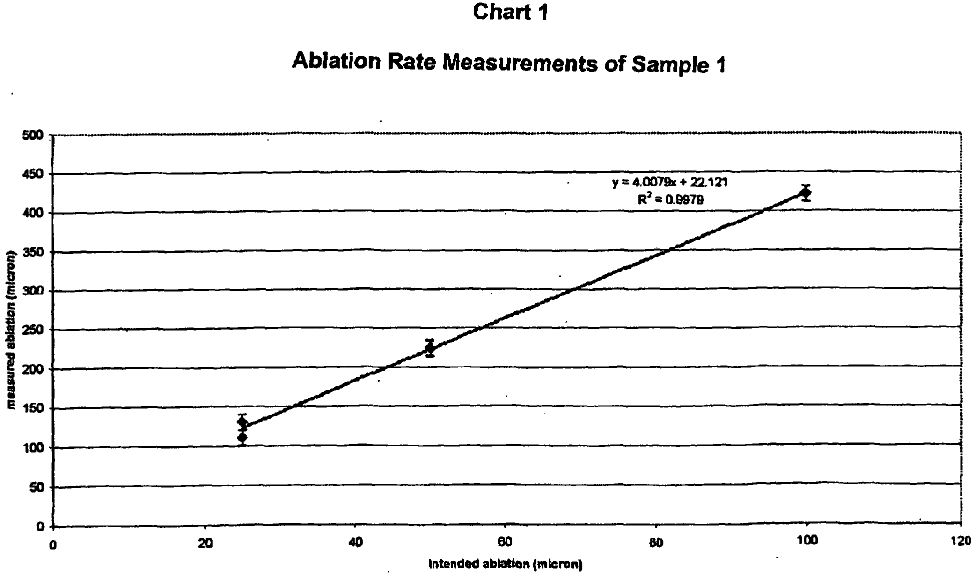

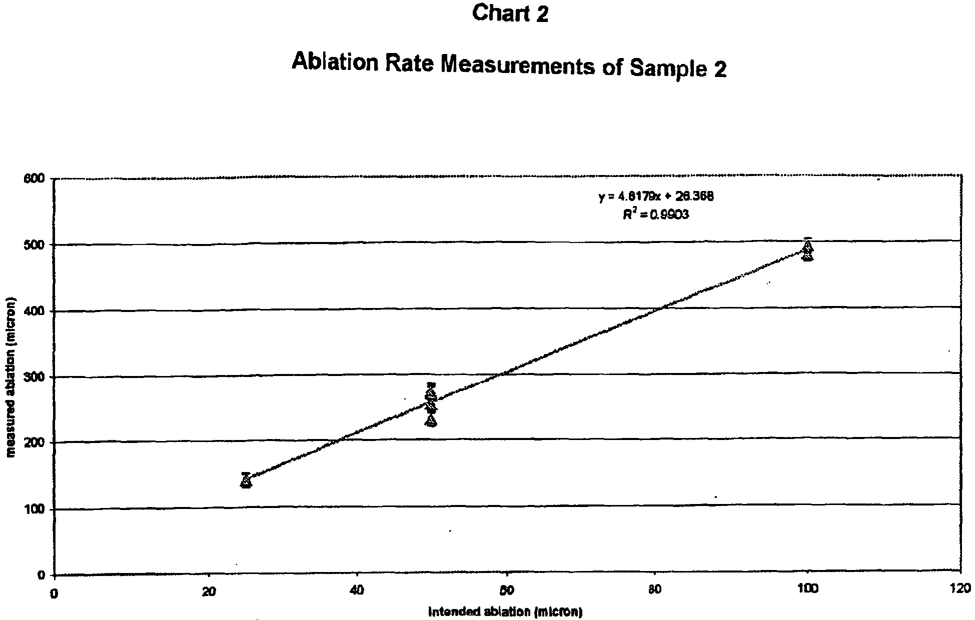

Upon observation of Samples 1 and 2, the ablation areas were clear with no signs of cracks or haze in both dark and bright field conditions. The unablated material however showed some haze when observed under dark field conditions. The ablation areas featured some striae and scattered vacuole-like features at 50 µm, and more noticeably at 100 µm, but not to a degree to cause a deleterious effect on vision. The cross-sectioned surfaces were rough at 100 µm but remained smooth at 25 and 50 µm when observed at x 20 magnification. The cross-sectioned surface of Sample 2 looked slightly smoother with less striation and deeper ablation than that of Sample 1 at 100 µm of intended ablation, possibly due to its higher water content.

PAls manufactured using the hydrogel polymeric materials are preferably of a round or oval design capable of being placed on the cornea of an eye under a cornea tissue flap made by a microkeratome or like surgical devices, or by like surgical methods known to those skilled in the art of ophthalmology. PAls of the present invention are manufactured by selecting the desired hydrogel polymeric material and cast molding the material using techniques known to those skilled in the art or casting the material as a film or rod. If cast as a film or rod, the material film or rod is then lathed or machined into a round or oval PAI. The PAls once manufactured are cleaned, polished, optionally hydrated, packaged and sterilized by customary methods known to those skilled in the art.

## Claims

1. A photoablatable cornea inlay manufactured from a composition comprising:
a copolymer of hydrophilic monomers, wherein said hydrophilic monomers are selected from the group consisting of 2-hydroxyethyl methacrylate, hydroxyethoxyethyl methacrylate, hydroxydiethoxyethyl methacrylate, methoxyethyl methacrylate, methoxyethoxyethyl methacrylate, methoxydiethoxyethyl methacrylate, poly(ethylene glycol) methacrylate, methoxy-poly(ethylene glycol) methacrylate, methacrylic acid, sodium methacrylate, glycerol methacrylate, hydroxypropyl methacrylate, N-vinylpyrrolidinone, hydroxypropyl methacrylamide, N,N-dimethylacrylamide, N-methylacrylamide and hydroxybutyl methacrylate;
a crosslinker; and
an initiator to form a hydrogel polymeric material with a water content of approximately 60 percent or greater by weight that shows no cracking or haze upon clinical photoablation, the hydrogel polymeric material being manufactured in the form of a disc or lenticule for placement in the corneal bed of an eye following surgical formation of a corneal flap.

2. The inlay of claim 1 wherein said composition includes an ultraviolet light absorbing material.

3. The inlay of claim 1 wherein said composition includes an ultraviolet light absorbing material selected from the group consisting of beta-(4-benzotriazoyl-3-hydroxyphenoxy)ethyl acrylate, 4-(2-acryloxyethoxy)-2-hydroxybenzophenone, 4-methacryloxy-2-hydroxybenzophenone, 2-(2'-methacryloxy-5'-methylphenyl)benzotriazole, 2-(2'-hydroxy-5'-methacryloxyethylphenyl)-2H-benzotriazole, 2-[3'-tert-butyl-2'-hydroxy-5'-(3"-methacryloyloxypropyl)phenyl]-5-chlorobenzotriazole, 2-[3'-*tert*-butyl-5'-(3-dimethylvinylsilylpropoxy)-2'-hydroxyphenyl]-5-methoxybenzotriazole, 2-(3'-allyl-2'-hydroxy-5'-methylphenyl)benzotriazole, 2-[3'-*tert*-butyl-2'-hydroxy-5'-(3"-methacryloyloxypropoxy)phenyl]-5-methoxybenzotriazole and 2-[3'-*tert*-butyl-2'-hydroxy-5'-(3"-methacryloyloxypropoxy)phenyl]-5-chlorobenzotriazole.

4. The inlay of claim 1 wherein said composition includes beta-(4-benzotriazoyl-3-hydroxyphenoxy)-ethyl acrylate as an ultraviolet light absorbing material.

5. The inlay of claim 1 wherein said initiator is selected from the group consisting of azobis(isobutyronitrile), 2,2'-azobis(2,4-dimethylvaleronitrile), 2,2'-azobis(methylbutyronitrile), 1,1'-azobis(cyanocyclohexane), di-t-butyl peroxide, dicumyl peroxide, t-butylcumyl peroxide, 2,5-dimethyl-2,5-bis(2-ethylhexanoylperoxy)hexane, t-butylperoxyneodecanote, t-butyl peroxy 2-ethylhexanoate, di(4-t-butyl cyclohexyl) peroxydicarbonate, t-butyl peroxypivalate, decanoyl peroxide, lauroyl peroxide, acetyl peroxide, stearoyl peroxide, benzoyl peroxide, 2,4-pentanedione peroxide, di(n-propyl)peroxydicarbonate, peroxydicarbonate, t-amyl peroxyneodecanoate, t-butyl peroxyacetate, benzoin methyl ether, and benzoin ethyl ether.

6. The inlay of claim 1 wherein said initiator is azobis(isobutyronitrile).

7. The inlay of claim 1 wherein said crosslinker is selected from the group consisting of ethylene glycol dimethacrylate, diethylene glycol dimethacrylate, triethylene glycol dimethacrylate and poly(ethylene glycol) dimethacrylate.

8. The inlay of claim 1 wherein said crosslinker is ethylene glycol dimethacrylate.

9. The inlay of claim 1 wherein the composition comprises 2-hydroxyethyl methacrylate or methacrylic acid as hydrophilic monomer.

10. The inlay of claim 1 wherein said crosslinker is ethylene glycol dimethacrylate, and the composition comprises 2-hydroxyethyl methacrylate or methacrylic acid as hydrophilic monomer.

11. The inlay of claim 1 wherein the water content is from 65% to 90% by weight.

12. The inlay of claim 1 wherein the water content is from 70% to 80% by weight.

## Patentansprüche

1. Ein durch Photoablation modifizierbares Hornhaut-Inlay, hergestellt aus einer Zusammensetzung, umfassend:
ein Copolymer aus hydrophilen Monomeren, wobei die hydrophilen Monomere ausgewählt sind aus der Gruppe bestehend aus 2-Hydroxyethylmethacrylat, Hydroxyethoxyethylmethacrylat, Hydroxydiethoxyethylmethacrylat, Methoxyethylmethacrylat, Methoxyethoxyethylmethacrylat, Methoxydiethoxyethylmethacrylat, Poly(ethylenglykol)methacrylat, Methoxy-poly(ethylenglykol)methacrylat, Methacrylsäure, Natriummethacrylat, Glycerinmethacrylat, Hydroxypropylmethacrylat, N-Vinylpyrrolidinon, Hydroxypropylmethacrylamid, N,N-Dimethylacrylamid,
N-Methylacrylamid und Hydroxybutylmethacrylat;
ein Vernetzungsmittel; und
einen Initiator, um ein polymeres Hydrogelmaterial mit einem Wassergehalt von ungefähr 60 Gew.-% oder mehr zu bilden, welches keine Rissbildung oder Eintrübung bei klinischer Photoablation aufweist, wobei das polymere Hydrogelmaterial in der Form einer Scheibe oder eines Lentikels zur Platzierung im Hornhautbett eines Auges nach operativer Bildung einer Hornhautklappe hergestellt ist.

2. Das Inlay gemäß Anspruch 1, wobei die Zusammensetzung ein ultraviolettes Licht absorbierendes Material einschließt.

3. Das Inlay gemäß Anspruch 1, wobei die Zusammensetzung ein ultraviolettes Licht absorbierendes Material, ausgewählt aus der Gruppe bestehend aus Beta-(4-benzo-triazoyl-3-hydroxyphenoxy)ethylacrylat, 4-(2-Acryloxyethoxy)-2-hydroxybenzophenon, 4-Methacryloxy-2-hydroxybenzophenon, 2-(2'-Methacryloxy-5'-methylphenyl)benzotriazol, 2-(2'-Hydroxy-5'-methacryloxyethylphenyl)-2H-benzotriazol, 2-[3'-tert-Butyl-2'-hydroxy-5'-(3"-methacryloyloxypropyl)phenyl]-5-chlorbenzotriazol, 2-[3'-*tert*-Butyl-5'-(3-dimethylvinylsilylpropoxy)-2'-hydroxyphenyl]-5-methoxybenzotriazol, 2-(3'-Allyl-2'-hydroxy-5'-methylphenyl)benzotriazol, 2-[3'-*tert*-Butyl-2'-hydroxy-5'-(3"-methacryloyl-oxypropoxy)phenyl]-5-methoxybenzotriazol und 2-[3'-*tert*-Butyl-2'-hydroxy-5'-(3"-methacryloyloxypropoxy)phenyl]-5-chlorbenzotriazol, einschließt.

4. Das Inlay gemäß Anspruch 1, wobei die Zusammensetzung Beta-(4-benzotriazoyl-3-hydroxyphenoxy)-ethylacrylat als ein ultraviolettes Licht absorbierendes Material einschließt.

5. Das Inlay gemäß Anspruch 1, wobei der Initiator ausgewählt ist aus der Gruppe bestehend aus Azobis(isobutyronitril), 2,2'-Azobis(2,4-dimethylvaleronitril), 2,2'-Azobis(methylbutyronitril), 1,1'-Azobis(cyanocyclohexan), Di-t-butylperoxid, Dicumylperoxid, t-Butylcumylperoxid, 2,5-Dimethyl-2,5-bis(2-ethylhexanoylperoxy)hexan, t-Butylperoxyneodecanoat, t-Butylperoxy-2-ethylhexanoat, Di(4-t-butyl-cyclohexyl)-peroxydicarbonat, t-Butylperoxypivalat, Decanoylperoxid, Lauroylperoxid, Acetylperoxid, Stearoylperoxid, Benzoylperoxid, 2,4-Pentandionperoxid, Di(n-propyl)peroxydicarbonat, Peroxydicarbonat, t-Amylperoxyneodecanoat, t-Butylperoxyacetat, Benzoinmethylether und Benzoinethylether.

6. Das Inlay gemäß Anspruch 1, wobei der Initiator Azobis(isobutyronitril) ist.

7. Das Inlay gemäß Anspruch 1, wobei das Vernetzungsmittel ausgewählt ist aus der Gruppe bestehend aus Ethylenglykoldimethacrylat, Diethylenglykoldimethacrylat, Triethylenglykoldimethacrylat und Poly(ethylenglykol)dimethacrylat.

8. Das Inlay gemäß Anspruch 1, wobei das Vernetzungsmittel Ethylenglykoldimethacrylat ist.

9. Das Inlay gemäß Anspruch 1, wobei die Zusammensetzung 2-Hydroxyethylmethacrylat oder Methacrylsäure als hydrophiles Monomer umfasst.

10. Das Inlay gemäß Anspruch 1, wobei das Vernetzungsmittel Ethylenglykoldimethacrylat ist und die Zusammensetzung 2-Hydroxyethylmethacrylat oder Methacrylsäure als hydrophiles Monomer umfasst.

11. Das Inlay gemäß Anspruch 1, wobei der Wassergehalt von 65 Gew.-% bis 90 Gew.-% beträgt.

12. Das Inlay gemäß Anspruch 1, wobei der Wassergehalt von 70 Gew.-% bis 80 Gew.-% beträgt.

## Revendications

1. Lenticule intra-cornéen photoablatif fabriqué à partir d'une composition comprenant :
un copolymère de monomères hydrophiles, dans lequel lesdits monomères hydrophiles sont choisis dans le groupe constitué du méthacrylate de 2-hydroxyéthyle, du méthacrylate d'hydroxyéthoxyéthyle, du méthacrylate d'hydroxydiéthoxyéthyle, du méthacrylate de méthoxyéthyle, du méthacrylate de méthoxyéthoxyéthyle, du méthacrylate de méthoxydiéthoxyéthyle, du méthacrylate de poly(éthylène glycol), du méthacrylate de méthoxy-poly(éthylène glycol), de l'acide méthacrylique, du méthacrylate de sodium, du méthacrylate de glycérol, du méthacrylate d'hydroxypropyle, de la N-vinylpyrrolidinone, du méthacrylamide d'hydroxypropyle, du N,N-diméthylacrylamide, du N-méthylacrylamide et du méthacrylate d'hydroxybutyle ;
un agent de réticulation ; et
un initiateur pour former un matériau polymère d'hydrogel ayant une teneur en eau d'environ 60 pour cent ou plus en poids qui ne présente aucun fendillement ou trouble suite à une photoablation clinique, le matériau polymère d'hydrogel étant fabriqué sous la forme d'un disque ou d'un lenticule destiné à être mis en place dans le lit cornéen d'un oeil suite à la formation chirurgicale d'un volet cornéen.

2. Lenticule selon la revendication 1 dans lequel ladite composition inclut un matériau absorbant la lumière ultraviolette.

3. Lenticule selon la revendication 1 dans lequel ladite composition inclut un matériau absorbant la lumière ultraviolette choisi dans le groupe constitué de l'acrylate de bêta-(4-benzotriazoyl-3-hydroxyphénoxy)éthyle, de la 4-(2-acryloxyéthoxy)-2-hydroxybenzophénone, de la 4-méthacryloxy-2-hydroxybenzophénone, du 2-(2'-méthacryloxy-5'-méthylphényl)benzotriazole, du 2-(2'-hydroxy-5'-méthacryloxyéthylphényl)-2H-benzotriazole, du 2-[3'-*tert*-butyl-2'-hydroxy-5'-(3"-méthacryloyloxypropyl])phényl]-5-chlorobenzotriazole, du 2-[3'-*tert*-butyl-5'-(3-diméthylvinylsilylpropoxy)-2'-hydroxyphényl]-5-méthoxybenzotriazole, du 2-(3'-allyl-2'-hydroxy-5'-méthylphényl)benzotriazole, du 2-[3'-*tert*-butyl-2'-hydroxy-5'-(3"-méthacryloyloxypropoxy)phényl]-5-méthoxybenzotriazole et du 2-[3'-*tert*-butyl-2'-hydroxy-5'-(3"-méthacryloyloxypropoxy)phényl]-5-chlorobenzotriazole.

4. Lenticule selon la revendication 1 dans lequel ladite composition inclut de l'acrylate de bêta-(4-benzotriazoyl-3-hydroxyphénoxy)-éthyle en tant que matériau absorbant la lumière ultraviolette.

5. Lenticule selon la revendication 1 dans lequel ledit initiateur est choisi dans le groupe constitué de l'azobis(isobutyronitrile), du 2,2'-azobis(2,4-diméthylvaléronitrile), du 2,2'-azobis(méthylbutyronitrile), du 1, l'-azobis(cyanocyclohexane), du peroxyde de di-t-butyle, du peroxyde de dicumyle, du peroxyde de t-butylcumyle, du 2,5-diméthyl-2,5-bis(2-éthylhexanoylperoxy)hexane, du t-butylperoxynéodécanoate, du t-butyl-peroxy-2-éthylhexanoate, du di(4-t-butyl-cyclohexyl)peroxydicarbonate, du t-butyl-peroxypivalate, du peroxyde de décanoyle, du peroxyde de lauroyle, du peroxyde d'acétyle, du peroxyde de stéaroyle, du peroxyde de benzoyle, du peroxyde de 2,4-pentanedione, du di(n-propyl)peroxydicarbonate, du peroxydicarbonate, du t-amyl-peroxynéodécanoate, du t-butyl-peroxyacétate, du méthyléther de benzoïne et de l'éthyléther de benzoïne.

6. Lenticule selon la revendication 1 dans lequel ledit initiateur est de l'azobis(isobutyronitrile).

7. Lenticule selon la revendication 1 dans lequel ledit agent de réticulation est choisi dans le groupe constitué du diméthacrylate d'éthylène glycol, du diméthacrylate de diéthylène glycol, du diméthacrylate de triéthylène glycol et du diméthacrylate de poly(éthylène glycol).

8. Lenticule selon la revendication 1 dans lequel ledit agent de réticulation est du diméthacrylate d'éthylène glycol.

9. Lenticule selon la revendication 1 dans lequel la composition comprend du méthacrylate de 2-hydroxyéthyle ou de l'acide méthacrylique en tant que monomère hydrophile.

10. Lenticule selon la revendication 1 dans lequel ledit agent de réticulation est du diméthacrylate d'éthylène glycol, et la composition comprend du méthacrylate de 2-hydroxyéthyle ou de l'acide méthacrylique en tant que monomère hydrophile.

11. Lenticule selon la revendication 1 dans lequel la teneur en eau est de 65 % à 90 % en poids.

12. Lenticule selon la revendication 1 dans lequel la teneur en eau est de 70 % à 80 % en poids.
